# EUROPEAN PATENT SPECIFICATION

(11) **EP 0 487 632 B1**
(45) Date of publication and mention of the grant of the patent: **04.03.1998**
(21) Application number: 90913372.0
(22) Date of filing: 14.08.1990
(51) Int. Cl.: A61K 39/12, A61K 39/13

(54) **STABILIZED VACCINE COMPOSITIONS**
STABILISIERTE IMPFSTOFFZUSAMMENSETZUNGEN
COMPOSITIONS DE VACCIN STABILISEES

(30) Priority: 15.08.1989 US 393996
(43) Date of publication of application: 03.06.1992
(73) Proprietor: MASSACHUSETTS INSTITUTE OF TECHNOLOGY, Cambridge, MA 02139 (US)
(72) Inventor: DORVAL, Brent, Dorchester, MA 02124 (US); CHOW, Marie, Brookline, MA 02146 (US); KLIBANOV, Alexander, Newton, MA 02159 (US)
(74) Representative: Kirkham, Nicholas Andrew
(86) International application number: PCT/US90/04581
(87) International publication number: WO 91/02543

(56) References cited:
- EP-A- 0 065 905
- GB-A- 1 564 998
- CHEMICAL ABSTRACTS, Volume 75, No. 5, 2 August 1971, (Columbus, Ohio, US), see page 283, Abstract 33217C, & JP,A,45 018 877 (Osaka University) 27 June 1970
- Biotechnology and Bioengineering, Volume 35, No. 4, April 1990, J. Wiley & Sons, Inc., (New York, N.Y., US), B.L. DORVAL et al.: "Lysine and other Diamines Dramatically Stabilize Poliovirus against Thermoinactivation" see pages 1051-1054
- Sons, Inc., (New York, N.Y., US), B.L. DORVAL et al.: "Lysine and other Diamines Dramatically Stabilize Poliovirus against Thermoinactivation" see pages 1051-1054
- Virology, vol. 6, 1958, p. 172-187
- Virology, vol. 15, 1961, p. 225-230

## Description

### Background of the Invention

The trivalent oral polio vaccine (Sabin) is a live-attenuated virus vaccine. It is heat-labile and hence must be stored frozen and used soon after thawing to insure effective immunization against poliomyelitis. Although 1 molar magnesium chloride is an effective stabilizer for the Sabin vaccine, inactivation will still occur if the vaccine thaws during transport or storage. Because of the shortage of adequate refrigeration facilities in under-developed and tropical regions, where poliovirus is endemic, the vaccine often cannot be stored frozen and as a consequence the vaccine becomes inactivated. This leads to under-immunization of the populations which are most at risk. Thus, eradication of poliomyelitis depends on the ability to assure cold storage and rapid distribution of poliovirus vaccine. Vaccine formulations with improved stability would circumvent this problem.

One class of stabilizing chemicals in live viral vaccine compositions is the amino acids. The basic amino acids were shown to have a stabilizing effect in live measles vaccine (M. Takanobu and T. Sasada, JP 70 18,877, June 27, 1970). L-cystine, in combination with tris(hydroxymethyl)aminomethane, was reported to stabilize poliovirus vaccine (Connaught Laboratories Limited, GB 1,564,998, April 16, 1980). The amino acids histidine, alanine, valine, threonine, arginine, methionine, hydroxyproline, lysine, isoleucine, phenylalanine, and serine were also shown to have stabilizing effectiveness in live viral vaccine compositions, with the preferred composition containing a combination of histidine and alanine (M. Barme, EP 0,065,905, May 10, 1982). However, these amino acid-containing vaccine compositions were demonstrated to be stabilizing at temperatures no higher than physiologic temperatures (36-37°C).

### Summary of the Invention

This invention pertains to stabilized viral vaccines, particularly live viral vaccines for poliomyelitis, comprising an aqueous solution of a live virus and a stabilizing amount of a compound containing at least two amino groups, such as basic amino acids (e.g. lysine). These compounds are safe, relatively inexpensive and can be easily added to viral vaccine preparations. The polyamino or -imine compound improves the heat stability of the virus in standard tests for viral stability over that of the currently available stabilizer magnesium chloride. This provides more stable live viral vaccine compositions for worldwide distribution and use.

According to the present invention there is provided a vaccine composition including a particular type of stabilizer and the use of the particular stabilizer as defined by the claims.

### Brief Description of the Figures

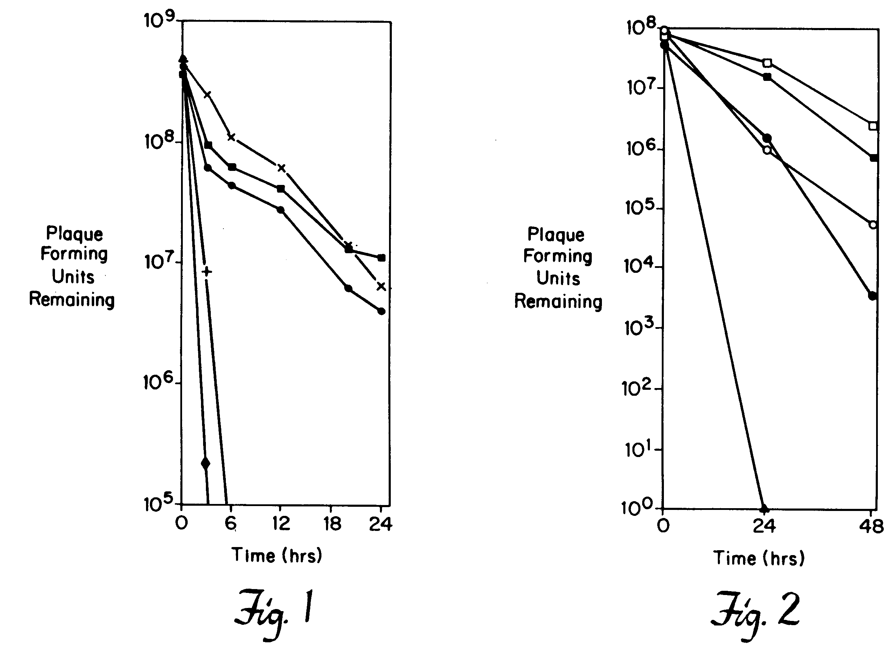

Figure 1 shows stabilization of poliovirus (serotype 1, Mahoney strain) against heat inactivation by 1 M amino acids or MgCl₂. Poliovirus 4 X 10⁸ plaque forming units (PFU, approximately 80 viral particles) was added to 1 ml of 5 mM phosphate buffer, pH 7.0, containing 1 M each of L-lysine (■), L-arginine (x), glycine ( 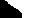 ), L-alanine (+) or MgCl₂ (●). The resultant solution was placed in 1.4 ml Eppendorf tubes, sealed and submerged in a water bath at 50 °C. Aliquots were removed periodically, diluted with with 5 mM phosphate buffer containing 150 mM NaCl, pH 7.0 (PBS), and the titer of infectious poliovirus was followed by plaque assay on HeLa cells.

Figure 2 shows stabilization of poliovirus (serotype 1, Mahoney strain) against heat inactivation by 1 or 2 M L-Lysine or MgCl₂. Poliovirus (8 X 10⁸ PFU) was added to 1 ml of 5 mM phosphate buffer, pH 7.5, alone (▲) or containing 1 M L-lysine (■), 2 M L-lysine (□), 1 M MgCl₂ (●) or 2 M MgCl₂ (○). The resulting solution was placed in 1.4 ml Eppendorf tubes, sealed and submerged in a water bath at 50 °C. Aliquots were removed periodically, diluted with PBS, and the titer of infectious poliovirus was followed by plaque assay on HeLa cells.

### Detailed Description of the Invention

The vaccine compositions of this invention comprise a virus and a compound which contains either at least two amino groups, or is a polyimine in a concentration of at least one molar. This compound enhances the stability of the virus against heat inactivation. For example, in standard tests for virus stability at 50°C, the stability of the virus is enhanced at least 10-20 fold by the amino acid lysine. The vaccine compositions are produced by adding the virus and the stabilizing amount (at least 1 molar) of the said amino compound or the polyimine into a physiologically acceptable aqueous solution.

The said compound can be any non-toxic compound containing at least two amino groups or is a polyimine. Preferably, the compounds comprise at least two primary or secondary amino groups separated by a spacer moiety. The size or constituency of the spacer moiety does not appear to be critical. Typically, the spacer moiety will consist of a substituted or unsubstituted, linear chain of carbon atoms (heteroatoms such as nitrogen may be included in the chain) ranging from 1 to about 10, preferably from 1 to about 6 atoms. The carbon chain may be saturated or unsaturated to various degrees. Preferred compounds are the basic amino acids lysine and arginine or salts (e.g., chloride or acetate) thereof. Some examples of other useful compounds include diaminoethane, 1,3-diaminopropane, 1,4-diaminobutane, and 1,5-diaminopentane. Other stabilizers include compounds which have a nitrogen carrying spacer moiety such as spermidine. Polyimines such as poly(ethylenimine) can also be used. Mixtures of stabilizer compounds can also be used.

The stabilizer compound is used in an amount effective to stabilize the virus and at a concentration of at least one molar. Generally, the concentration of the compound is 1-2 molar.

The virus can be any virus or mixture of viruses. Examples of such viruses include picornaviruses, such as polio virus; rotavirus; respiratory syncytial virus; measles virus; and rubella. Generally, the virus will be attenuated. For vaccines against poliomyelitis, the vaccine compositions can contain any or all of the various types of poliovirus. The preferred vaccines are the trivalent Sabin vaccines which contain types I, II and III of poliovirus.

The vaccine compositions will typically be formulated at a pH ranging from about 6 to 8. Magnesium chloride, preferably 1 molar, can also be added to the compositions.

Other immunogens such as diphtheria toxoid, tetanus toxoid and inactivated pertussis cells can combine with the viral components of the compositions. In addition, the compositions can contain adjuvants which do not interfere with the activity of the stabilizing compound.

The invention is illustrated further by the following exemplification. Although the stabilizing effects of the present invention are exemplified by a picornavirus, the present invention is useful to stabilize other viruses as well, including, but not limited to, those referred to above.

### Exemplification

### Methods and Materials

Poliovirus (serotype l, Mahoney strain) (PV1M) was grown in HeLa cells, purified on cesium chloride gradients and dialyzed against PBS, pH 7.2. Viral stocks contained approximately 4 x 10¹¹ PFU/ml and were stored at 4 °C.

Approximately 4 x 10⁸ PFU were added to 1 ml of 5 mM phosphate buffer, pH 7.0, alone or containing 1 M L-lysine, D-lysine, L-arginine, glycine, L-alanine, N-a-acetyl-L-lysine, N-e-acetyl-L-lysine, L-lysine methyl ester, ethylenediamine, 1,5-diaminopentane, ethylamine, poly(ethylenimine), spermidine or MgCl₂. The pH of each solution was adjusted to 7.0 with HCl prior to the addition of poliovirus. The resulting solutions were placed in 1.4 ml Eppendorf tubes, sealed and submerged in a water bath at 50 °C. Aliquots (10-100 ul) were removed periodically, diluted with PBS and the titer of infectious poliovirus was followed by plaque assay on HeLa cells.

### Results

In the first experiment the ability of 1 M concentrations of L-amino acids and MgCl₂, pH 7.0, to stabilize PV1M against heat inactivation at 50 °C was tested. Figure 1 demonstrates that lysine and arginine stabilize PV1M 2 to 4 times better than MgCl₂ at all time points, whereas, L-alanine and glycine provide 10 to 10,000 times less stabilization than MgCl₂ during the same period. In controls, which contained 5 mM phosphate buffer alone at pH 7.0, more than eight orders of magnitude of viral infectivity were lost after 3 hours.

Lysine concentrations fo 0.1 to 2 M were used to optimize PV1M stability. These data show that 0.3 M L-lysine or below provide little extra stability, and that at 2 M lysine poliovirus stability is maximal. Figure 2 compares stabilization of PVIM by 1 and 2 M L-lysine and MgCl₂ at pH 7.0. These data show that L-lysine is 10 and 20 times better than MgCl₂ at stabilizing PVIM after 24 and 48 hours, respectively, at 50°C.

Whether stabilization of PV1M by lysine is stereospecific was also assessed. To do this, L-and D-lysine were tested at 1 M concentrations. These data demonstrate that both L- and D-lysine are equally effective in stabilizing PV1M against heat inactivation at 50°C (Table 1).

**TABLE 1**

| TABLE 1. The effect of 1 M L- and D-lysine stereosomers on the stabilization of poliovirus (serotype 1, Mahoney strain) against heat inactivation^{a}. | | | |
|---|---|---|---|
| Time (hours at 50 °C) | Plaque Forming Units Remaining^{b} | | |
| | L-lysine | D-lysine | MgCl₂ |
| 0 | 3.7 | 5.9 | 4.1 |
| 3 | 2.1 | 3.1 | 0.61 |
| 6 | 0.93 | 0.82 | 0.44 |
| 12 | 0.65 | 0.49 | 0.28 |
| 20 | 0.13 | 0.09 | 0.06 |
| 24 | 0.11 | 0.05 | 0.04 |

| | | | |
|---|---|---|---|
| ^{a} Poliovirus (approximately 4 X 10⁸ PFU) was added to 1 ml of 5 mM phosphate buffer, pH 7.0 containing 1 M of the above compounds. The resulting solutions were placed in 1.4 ml Eppendorf tubes, sealed and submerged in a water bath at 50 °C. Aliquots were removed periodically, diluted with PBS and the titer of infectious poliovirus was followed by plaque assay on HeLa cells. | | | |
| ^{b} Values should be multiplied by 10⁸ | | | |

Since lysine has an a- and e-amino group which may be involved simultaneously in binding opposite charges on the capsid surface, the effect of a- or e-acetylated derivatives of L-lysine which lack the corresponding a- or e- NH₂ group was tested. In addition, the effect of the carboxyl group of L-lysine was tested by using L-lysine methyl ester. These data demonstrate that L-lysine or its methyl ester were equally protective against heat inactivation, whereas removal of either the a- or e-NH₂ group from L-lysine abbrogated the ability of these compounds to stabilize PV1M (Table 2).

**TABLE 2**

| TABLE 2. The effect of lysine modification on the stabilization of poliovirus (serotype 1, Mahoney strain) against heat inactivation^{a}. | | | | |
|---|---|---|---|---|
| Time (hours at 50 °C) | Plaque Forming Units Remaining^{b} | | | |
| | N-e-acetyl -L-lysine | N-a-acetyl -L-lysine | L-lysine methyl ester | lysine |
| 0 | 2.2 | 3.1 | 4.1 | 3.7 |
| 3 | 0.0005 | - | 1.1 | 2.1 |
| 6 | 0.00005 | - | 0.85 | 0.93 |
| 12 | -^{c} | - | 0.5 | 0.65 |
| 20 | - | - | 0.2 | 0.13 |
| 24 | - | - | 0.11 | 0.11 |

| | | | | |
|---|---|---|---|---|
| ^{a} Poliovirus (approximately 4 X 10⁸ PFU) was added to 1 ml of 5 mM phosphate buffer, pH 7.0 containing 1 M of the above compounds. The resulting solutions were placed in 1.4 ml Eppendorf tubes, sealed and submerged in a water bath at 50 °C. Aliquots were removed periodically, diluted with PBS and the titer of infectious poliovirus was followed by plaque assay on HeLa cells. | | | | |
| ^{b} Values should be multiplied by 10⁸. | | | | |
| ^{c} Values are below 100 PFU/ml. | | | | |

These data suggested that compounds other than lysine which contain 2 amino groups might be effective stabilizers.

Consequently, ethylenediamine, poly(ethylenimine), spermidine, 1-5 diaminopentane or ethylamine (a monoamine) were tested at 1 M concentration. These data show that ethylenediamine, 1-5 diaminopentane, poly(ethylenimine) are as effective, and spermidine is slightly less effective, than lysine at stabilizing PVIM, whereas ethylamine does not stabilize PV1M (Table 3).

**TABLE 3**

| TABLE 3. The effect of mono-, di- and polyamines and polyimines on the stabilization of poliovirus (serotype 1, Mahoney strain) against heat inactivation^{a}. | | | | | | |
|---|---|---|---|---|---|---|
| Time (hours at 50 °C) | Plaque Forming Units Remaining^{b} | | | | | |
| | ethylenediamine | poly(ethylen-imine) | ethylamine | spermidine | lysine | 1,5-diaminopentane |
| 0 | 5.2 | 2.3 | 3.9 | 4.1 | 3.7 | 4.3 |
| 3 | 2.2 | 2.6 | -^{c} | 0.19 | 2.1 | 1.4 |
| 6 | 0.6 | 1.7 | - | 0.25 | 0.93 | 1.5 |
| 12 | 0.75 | 0.72 | - | 0.19 | 0.65 | 0.79 |
| 20 | 0.2 | 0.14 | - | 0.041 | 0.13 | 0.25 |
| 24 | 0.19 | 0.07 | - | 0.034 | 0.11 | 0.13 |

| | | | | | | |
|---|---|---|---|---|---|---|
| ^{a} Poliovirus (approximately 4 X 10⁸ PFU) was added to 1 ml of 5 mM phosphate buffer, pH 7.0 containing 1 M of the above compounds. The resulting solutions were placed in 1.4 ml Eppendorf tubes, sealed and submerged in a water bath at 50 °C. Aliquots were removed periodically, diluted with PBS and the titer of infectious poliovirus was followed by plaque assay on HeLa cells. | | | | | | |
| ^{b} Values should be multiplied by 10⁸. | | | | | | |
| ^{c} Values are below 100 PFU/ml. | | | | | | |

## Claims (Claims for the following Contracting State(s): AT, BE, CH, LI, DE, DK, FR, GB, IT, LU, NL, SE)

1. A stabilized vaccine composition comprising:
a) a physiologically acceptable aqueous solution;
b) a virus; and
c) a stabilizer consisting of either a compound with at least two amino groups, or a polyimine, said stabilizer being present in the vaccine composition at a concentration of at least 1 Molar to stabilize the virus against heat inactivation.

2. A vaccine composition according to claim 1, wherein the virus is poliovirus, which may be attenuated.

3. A vaccine composition according to claim 2, wherein the polio virus comprises all three types I, II and III.

4. A vaccine composition according to any one of claims 1 to 3, wherein the stabilizer compound comprises two amino groups separated by a spacer moiety which is a substituted or unsubstituted linear chain of carbon atoms.

5. A vaccine composition according to claim 4, wherein the substituted linear chain of carbon atoms contains nitrogen.

6. A vaccine composition according to claim 1, wherein the stabilizer compound is selected from diaminoethane, diaminopropane, 1, 4-diaminobutane, 1, 5-diaminopentane, spermidine and poly(ethylenimine).

7. A vaccine composition according to claim 1, wherein the stabilizer compound is a basic amino acid or a salt thereof.

8. A vaccine composition according to claim 7, wherein the amino acid is lysine or arginine, or the methyl ester of lysine.

9. A vaccine composition according to claim 1, further comprising an immunogen selected from:
a) diphtheria toxoid;
b) tetanus toxoid; and
c) inactivated pertussis cells.

10. A vaccine composition according to claim 1, further including magnesium chloride, preferably at a 1 Molar concentration.

11. Use of a stabilizer to a virus in a vaccine composition, wherein the stabilizer consists of either a compound with at least two amino groups, or a polyimine, and the stabilizer is used at a concentration of at least 1 Molar to stabilize the vaccine composition.

12. The use according to claim 11, wherein the stabilizer compound comprises two amino groups separated by a spacer moiety which is a substituted or unsubstituted linear chain of carbon atoms.

13. The use according to claim 12, wherein the substituted linear chain of carbon atoms contains nitrogen.

14. The use according to claim 11, wherein the stabilizer compound is selected from the group consisting of: diaminoethane; diaminopropane; 1, 4-diaminobutane; 1, 5-diaminopentane; spermidine; poly(ethylenimine); a basic amino acid; a salt of a basic amino acid; and the methyl ester of lysine.

15. The use according to claim 11, wherein the stabilizer is for use in conjunction with an immunogen selected from:
a) diphtheria toxoid;
b) tetanus toxoid; and
c) inactivated pertussis cells.

16. The use according to claim 11, wherein the stabilizer is used in conjunction with magnesium chloride, preferably at a 1 Molar concentration.

17. The use according to any one of the preceding claims, wherein the stabilizer is used for stabilizing poliovirus, which may be attenuated and may comprise all three types I, II and III.

## Claims (Claims for the following Contracting State(s): ES)

1. A method of producing stabilized vaccine composition comprising the steps of providing:
a) a physiologically acceptable aqueous solution;
b) a virus; and
c) a stabilizer consisting of either a compound with at least two amino groups, or a polyimine, said stabilizer being present in the vaccine composition at a concentration of at least 1 Molar to stabilize the virus against heat inactivation.

2. A method according to claim 1, wherein the virus is poliovirus, which may be attenuated.

3. A method according to claim 2, wherein the polio virus comprises all three types I, II and III.

4. A method according to any one of claims 1 to 3, wherein the stabilizer compound comprises two amino groups separated by a spacer moiety which is a substituted or unsubstituted linear chain of carbon atoms.

5. A method according to claim 4, wherein the substituted linear chain of carbon atoms contains nitrogen.

6. A method according to claim 1, wherein the stabilizer compound is selected from diaminoethane, diaminopropane, 1, 4-diaminobutane, 1, 5-diaminopentane, spermidine and poly(ethylenimine).

7. A method according to claim 1, wherein the stabilizer compound is a basic amino acid or a salt thereof.

8. A method according to claim 7, wherein the amino acid is lysine or arginine, or the methyl ester of lysine.

9. A method according to claim 1, further comprising an immunogen selected from:
a) diphtheria toxoid;
b) tetanus toxoid; and
c) inactivated pertussis cells.

10. A method according to claim 1, further including magnesium chloride, preferably at a 1 Molar concentration.

11. Use of a stabilizer to a virus in a vaccine composition, wherein the stabilizer consists of either a compound with at least two amino groups, or a polyimine, and the stabilizer is used at a concentration of at least 1 Molar to stabilize the vaccine composition.

12. The use according to claim 11, wherein the stabilizer compound comprises two amino groups separated by a spacer moiety which is a substituted or unsubstituted linear chain of carbon atoms.

13. The use according to claim 12, wherein the substituted linear chain of carbon atoms contains nitrogen.

14. The use according to claim 11, wherein the stabilizer compound is selected from the group consisting of: diaminoethane; diaminopropane; 1, 4-diaminobutane; 1, 5-diaminopentane; spermidine; poly(ethylenimine); a basic amino acid; a salt of a basic amino acid; and the methyl ester of lysine.

15. The use according to claim 11, wherein the stabilizer is for use in conjunction with an immunogen selected from:
a) diphtheria toxoid;
b) tetanus toxoid; and
c) inactivated pertussis cells.

16. The use according to claim 11, wherein the stabilizer is used in conjunction with magnesium chloride, preferably at a 1 Molar concentration.

17. The use according to any one of the preceding claims, wherein the stabilizer is used for stabilizing poliovirus, which may be attenuated and may comprise all three types I, II and III.

## Patentansprüche (Patentansprüche für folgende(n) Vertragsstaat(en): AT, BE, CH, LI, DE, DK, FR, GB, IT, LU, NL, SE)

1. Eine stabilisierte Impfstoffzusammensetzung, die umfaßt:
a) eine physiologisch geeignete wäßrige Lösung;
b) ein Virus; und
c) einen Stabilisator, der entweder aus einer Verbindung mit mindestens zwei Aminogruppen oder aus einem Polyimin besteht, wobei besagter Stabilisator in der Impfstoffzusammensetzung in einer mindestens 1 molaren Konzentration vorhanden ist, um das Virus gegen Hitzeinaktivierung zu stabilisieren.

2. Eine Impfstoffzusammensetzung gemäß Anspruch 1, worin das Virus Polio-Virus ist, das attenuiert sein kann.

3. Eine Impfstoffzusammensetzung gemäß Anspruch 2, worin das Polio-Virus alle drei Typen I, II und III umfaßt.

4. Eine Impfstoffzusammensetzung gemäß einem der Ansprüche 1 bis 3, worin die Stabilisatorverbindung zwei Aminogruppen umfaßt, die durch ein Brückenglied getrennt sind, das eine substituierte oder nicht substituierte unverzweigte Kette aus Kohlenstoffatomen ist.

5. Eine Impfstoffzusammensetzung gemäß Anspruch 4, worin die substituierte unverzweigte Kette aus Kohlenstoffatomen Stickstoff enthält.

6. Eine Impfstoffzusammensetzung gemäß Anspruch 1, worin die Stabilisatorverbindung aus Diaminoethan, Diaminopropan, 1,4-Diaminobutan, 1,5-Diaminopentan, Spermidin und Poly(ethylenimin) ausgewählt ist.

7. Eine Impfstoffzusammensetzung gemäß Anspruch 1, worin die Stabilisatorverbindung eine basische Aminosäure oder ein Salz davon ist.

8. Eine Impfstoffzusammensetzung gemäß Anspruch 7, worin die Aminosäure Lysin oder Arginin oder der Methylester von Lysin ist.

9. Eine Impfstoffzusammensetzung gemäß Anspruch 1, die außerdem ein Immunogen umfaßt, das ausgewählt ist aus:
a) Diphtherietoxoid;
b) Tetanustoxoid; und
c) inaktivierten Pertussis-Zellen.

10. Eine Impfstoffzusammensetzung gemäß Anspruch 1, die außerdem Magnesiumchlorid, vorzugsweise in einer 1 molaren Konzentration, enthält.

11. Verwendung eines Stabilisators für ein Virus in einer Impfstoffzusammensetzung, worin der Stabilisator entweder aus einer Verbindung mit mindestens zwei Aminogruppen oder aus einem Polyimin besteht und der Stabilisator in mindestens einer 1 molaren Konzentration verwendet wird, um die Impfstoffzusammensetzung zu stabilisieren.

12. Die Verwendung gemäß Anspruch 11, worin die Stabilisatorverbindung zwei Aminogruppen umfaßt, die durch ein Brückenglied getrennt sind, das eine substituierte oder nicht substituierte unverzweigte Kette aus Kohlenstoffatomen ist.

13. Die Verwendung gemäß Anspruch 12, worin die substituierte unverzweigte Kette aus Kohlenstoffatomen Stickstoff enthält.

14. Die Verwendung gemäß Anspruch 11, worin die Stabilisatorverbindung aus der Gruppe ausgewählt ist bestehend aus: Diaminoethan; Diaminopropan; 1,4-Diaminobutan; 1,5-Diaminopentan; Spermidin; Poly(ethylenimin); einer basischen Aminosäure; einem Salz einer basischen Aminosäure; und dem Methylester von Lysin.

15. Die Verwendung gemäß Anspruch 11, worin der Stabilisator zusammen mit einem Immunogen verwendet werden kann, das ausgewählt ist aus:
a) Diphtherietoxoid;
b) Tetanustoxoid; und
c) inaktivierten Pertussis-Zellen.

16. Die Verwendung gemäß Anspruch 11, worin der Stabilisator zusammen mit Magnesiumchlorid, vorzugsweise in einer 1 molaren Konzentration, verwendet ist.

17. Die Verwendung gemäß einem der vorausgehenden Ansprüche, worin der Stabilisator zur Stabilisierung eines Polio-Virus, das attenuiert sein kann und alle drei Typen I, II und III umfassen kann, verwendet ist.

## Patentansprüche (Patentansprüche für folgende(n) Vertragsstaat(en): ES)

1. Verfahren zur Herstellung einer stabilisierten Impfstoffzusammensetzung, das die Bereitstellungsschritte umfaßt:
a) eine physiologisch geeignete wäßrige Lösung;
b) ein Virus; und
c) einen Stabilisator, der entweder aus einer Verbindung mit mindestens zwei Aminogruppen oder aus einem Polyimin besteht, wobei besagter Stabilisator in der Impfstoffzusammensetzung in einer mindestens 1 molaren Konzentration vorhanden ist, um das Virus gegen Hitzeinaktivierung zu stabilisieren.

2. Ein Verfahren gemäß Anspruch 1, worin das Virus Polio-Virus ist, das attenuiert sein kann.

3. Ein Verfahren gemäß Anspruch 2, worin das Polio-Virus alle drei Typen I, II und III umfaßt.

4. Ein Verfahren gemäß einem der Ansprüche 1 bis 3, worin die Stabilisatorverbindung zwei Aminogruppen umfaßt, die durch ein Brückenglied getrennt sind, das eine substituierte oder nicht substituierte unverzweigte Kette aus Kohlenstoffatomen ist.

5. Ein Verfahren gemäß Anspruch 4, worin die substituierte unverzweigte Kette aus Kohlenstoffatomen Stickstoff enthält.

6. Ein Verfahren gemäß Anspruch 1, worin die Stabilisatorverbindung aus Diaminoethan, Diaminopropan, 1,4-Diaminobutan, 1,5-Diaminopentan, Spermidin und Poly(ethylenimin) ausgewählt ist.

7. Ein Verfahren gemäß Anspruch 1, worin die Stabilisatorverbindung eine basische Aminosäure oder ein Salz davon ist.

8. Ein Verfahren gemäß Anspruch 7, worin die Aminosäure Lysin oder Arginin oder der Methylester von Lysin ist.

9. Ein Verfahren gemäß Anspruch 1, das außerdem ein Immunogen umfaßt, das ausgewählt ist aus:
a) Diphtherietoxoid;
b) Tetanustoxoid; und
c) inaktivierten Pertussis-Zellen.

10. Ein Verfahren gemäß Anspruch 1, das außerdem Magnesiumchlorid, vorzugsweise in einer 1 molaren Konzentration, umfaßt.

11. Verwendung eines Stabilisators für ein Virus in einer Impfstoffzusammensetzung, worin der Stabilisator entweder aus einer Verbindung mit mindestens zwei Aminogruppen oder aus einem Polyimin besteht und der Stabilisator in mindestens einer 1 molaren Konzentration verwendet wird, um die Impfstoffzusammensetzung zu stabilisieren.

12. Die Verwendung gemäß Anspruch 11, worin die Stabilisatorverbindung zwei Aminogruppen umfaßt, die durch ein Brückenglied getrennt sind, das eine substituierte oder nicht substituierte unverzweigte Kette aus Kohlenstoffatomen ist.

13. Die Verwendung gemäß Anspruch 12, worin die substituierte unverzweigte Kette aus Kohlenstoffatomen Stickstoff enthält.

14. Die Verwendung gemäß Anspruch 11, worin die Stabilisatorverbindung aus der Gruppe ausgewählt ist bestehend aus: Diaminoethan; Diaminopropan; 1,4-Diaminobutan; 1,5-Diaminopentan; Spermidin; Poly(ethylenimin); einer basischen Aminosäure; einem Salz einer basischen Aminosäure; und dem Methylester von Lysin.

15. Die Verwendung gemäß Anspruch 11, worin der Stabilisator zusammen mit einem Immunogen verwendet werden kann, das ausgewählt ist aus:
a) Diphtherietoxoid;
b) Tetanustoxoid; und
c) inaktivierten Pertussis-Zellen.

16. Die Verwendung gemäß Anspruch 11, worin der Stabilisator zusammen mit Magnesiumchlorid, vorzugsweise in einer 1 molaren Konzentration, verwendet ist.

17. Die Verwendung gemäß einem der vorausgehenden Ansprüche, worin der Stabilisator zur Stabilisierung eines Polio-Virus, das attenuiert sein kann und alle drei Typen I, II und III umfassen kann, verwendet ist.

## Revendications (Revendications pour l'(les) Etat(s) contractant(s) suivant(s): AT, BE, CH, LI, DE, DK, FR, GB, IT, LU, NL, SE)

1. Composition vaccinale stabilisée comprenant
a) une solution aqueuse physiologiquement acceptable;
b) un virus; et
c) un stabilisant consistant, soit en un composé portant au moins deux groupes amino, soit en une polyimine, ledit stabilisant étant présent dans la composition vaccinale à une concentration d'au moins 1 molaire pour stabiliser le virus vis-à-vis de l'inactivation par la chaleur.

2. Composition vaccinale selon la revendication 1, dans laquelle le virus est un virus poliomyélitique qui peut être atténué.

3. Composition vaccinale selon la revendication 2, dans laquelle le virus poliomyélitique comprend les trois types, I, II et III.

4. Composition vaccinale selon l'une des revendications 1 à 3, dans laquelle l'agent stabilisant comprend des groupes amino séparés par un espaceur qui est une chaîne linéaire d'atomes de carbone substituée ou non.

5. Composition vaccinale selon la revendication 4, dans laquelle la chaîne linéaire d'atomes de carbone substituée contient de l'azote.

6. Composition vaccinale selon la revendication 1, dans laquelle l'agent stabilisant est choisi parmi le diaminoéthane, le diaminopropane, le 1,4 diaminobutane, le 1,5-diaminopentane, la spermidine et la poly(éthylènimine).

7. Composition vaccinale selon la revendication 1, dans laquelle l'agent stabilisant est un acide aminé basique ou un sel de ce dernier.

8. Composition vaccinale selon la revendication 7, dans laquelle l'acide aminé est la lysine ou l'arginine ou l'ester méthylique de lysine.

9. Composition vaccinale selon la revendication 1, comprenant de surcroît un agent immunogène choisi entre :
a) la toxine diphtérique;
b) la toxine tétanique; et
c) des bacilles coquelucheux inactivés .

10. Composition vaccinale selon la revendication 1, comprenant de surcroît du chlorure de magnésium, de préférence à une concentration de 1 molaire.

11. Utilisation dans une composition vaccinale d'un agent stabilisant un virus, dans laquelle l'agent stabilisant consiste en un composé portant au moins deux groupes amino, ou en une polyimine, ledit agent stabilisant étant utilisé à une concentration d'au moins 1 molaire pour stabiliser la composition vaccinale.

12. Utilisation selon la revendication 11, dans laquelle l'agent stabilisant comprend deux groupes amino séparés par un espaceur qui est une chaîne linéaire d'atomes de carbone substituée ou non.

13. Utilisation selon la revendication 12, dans laquelle la chaîne linéaire d'atomes de carbone substituée contient de l'azote.

14. Utilisation selon la revendication 11, dans laquelle l'agent stabilisant est choisi au sein du groupe constitué par le diaminoéthane; le diaminopropane; le 1,4 diaminobutane; le 1,5-diaminopentane; la spermidine; la poly(éthylènimine); un acide aminé basique; un sel d'acide aminé basique; et l'ester méthylique de lysine.

15. Utilisation selon la revendication 11, dans laquelle l'agent stabilisant est utilisé en association avec un agent immunogène choisi parmi :
a) la toxine diphtérique;
b) la toxine tétanique; et
c) des bacilles coquelucheux inactivés .

16. Utilisation selon la revendication 11, dans laquelle l'agent stabilisant est utilisé en association avec du chlorure de magnésium, de préférence à une concentration de 1 molaire.

17. Utilisation selon l'une quelconque des revendications précédentes, dans laquelle l'agent stabilisant est utilisé pour stabiliser le virus poliomyélitique, qui peut être atténué et peut comprendre les trois types I, II et III.

## Revendications (Revendications pour l'(les) Etat(s) contractant(s) suivant(s): ES)

1. Procédé de préparation d'une composition vaccinale stabilisée comprenant les étapes consistant à fournir :
a) une solution aqueuse physiologiquement acceptable ;
b) un virus ; et
c) un stabilisant consistant, soit en un composé portant au moins deux groupes amino, soit en une polyimine, ledit stabilisant étant présent dans la composition vaccinale à une concentration d'au moins 1 molaire pour stabiliser le virus vis-à-vis de l'inactivation par la chaleur.

2. Procédé selon la revendication 1, dans lequel le virus est un virus poliomyélitique qui peut être atténué.

3. Procédé selon la revendication 2, dans lequel le virus poliomyélitique comprend les trois types I, II et III.

4. Procédé selon l'une des revendications 1 à 3, dans lequel l'agent stabilisant comprend deux groupes amino séparés par un espaceur qui est une chaîne linéaire d'atomes de carbone substituée ou non.

5. Procédé selon la revendication 4, dans lequel la chaîne linéaire d'atomes de carbone substituée contient de l'azote.

6. Procédé selon la revendication 1, dans lequel l'agent stabilisant est choisi parmi le diaminoéthane, le diaminopropane, le 1,4 diaminobutane, le 1,5-diaminopentane, la spermidine et la poly(éthylènimine).

7. Procédé selon la revendication 1, dans lequel l'agent stabilisant est un acide aminé basique ou un sel de ce dernier.

8. Procédé selon la revendication 7, dans lequel l'acide aminé est la lysine ou l'arginine ou l'ester méthylique de lysine.

9. Procédé selon la revendication 1, comprenant de surcroît un agent immunogène choisi entre :
a) la toxine diphtérique ;
b) la toxine tétanique ;
c) des bacilles coquelucheux inactivés.

10. Procédé selon la revendication 1, comprenant de surcroît du chlorure de magnésium, de préférence à une concentration de 1 molaire.

11. Utilisation dans une composition vaccinale d'un agent stabilisant un virus, dans laquelle l'agent stabilisant consiste en un composé portant au moins deux groupes amino, ou en une polyimine, ledit agent stabilisant étant utilisé à une concentration d'au moins 1 molaire pour stabiliser la composition vaccinale.

12. Utilisation selon la revendication 11, dans laquelle l'agent stabilisant comprend deux groupes amino séparés par un espaceur qui est une chaîne linéaire d'atomes de carbone substituée ou non.

13. Utilisation selon la revendication 12, dans laquelle la chaîne linéaire d'atomes de carbone substituée contient de l'azote.

14. Utilisation selon la revendication 11, dans laquelle l'agent stabilisant est choisi au sein du groupe constitué par le diaminoéthane ; le diaminopropane ; le 1,4 diaminobutane ; le 1,5-diaminopentane ; la spermidine ; la poly(éthylènimine) ; un acide aminé basique ; un sel d'acide aminé basique ; et l'ester méthylique de lysine.

15. Utilisation selon la revendication 11, dans laquelle l'agent stabilisant est utilisé en association avec un agent immunogène choisi parmi :
a) la toxine diphtérique ;
b) la toxine tétanique ; et
c) des bacilles coquelucheux inactivés.

16. Utilisation selon la revendication 11, dans laquelle l'agent stabilisant est utilisé en association avec du chlorure de magnésium, de préférence à une concentration de 1 molaire.

17. Utilisation selon l'une quelconque des revendications précédentes, dans laquelle l'agent stabilisant est utilisé pour stabiliser le virus poliomyélitique, qui peut être atténué et peut comprendre les trois types I, II et III.
